# EUROPEAN PATENT SPECIFICATION

(11) **EP 2 662 015 B1**
(45) Date of publication and mention of the grant of the patent: **27.04.2016**
(21) Application number: 13167131.5
(22) Date of filing: 08.05.2013
(51) Int. Cl.: A61B 1/00, A61B 5/06, A61B 18/14, A61M 25/00, A61B 5/00, A61M 25/01, A61B 18/00

(54) **LOCATING A CATHETER SHEATH END POINT**
Lokalisierung eines Katheterhüllenendpunkts
Localisation d'un point d'extrémité de gaine de cathéter

(30) Priority: 09.05.2012 US 201213467158
(43) Date of publication of application: 13.11.2013
(73) Proprietor: Biosense Webster (Israel), Ltd., Yokneam 20692 (IL)
(72) Inventor: Ludwin, Doron Moshe, 32207 Haifa (IL); Bar-Tal, Meir, 32240 Haifa (IL); Turgemann, Aharon, 30900 Zichron Ya'acov (IL)
(74) Representative: Carpmaels & Ransford LLP

(56) References cited:
- WO-A1-00/19917
- WO-A2-2007/103362
- US-A1- 2009 138 007
- US-B1- 6 911 026

## Description

### FIELD OF THE INVENTION

The present invention relates generally to invasive medical procedures, and specifically to invasive insertion of a probe into a sheath guiding the probe.

### BACKGROUND OF THE INVENTION

In a number of medical procedures wherein a probe is inserted into a patient, the probe is inserted through a sheath. Typically the sheath acts to guide the probe during its insertion, as well as to maintain the probe in a desired alignment. Once the probe and the sheath have been inserted into the patient, their distal ends are not visible, so that an operator performing the procedure may be unaware of undesired overlap of the sheath distal end relative to the probe distal end.

United States patent application publication number US 2009/0138007 A1, on which the preamble of claim 1 is based, describes a medical probe including a and insertion tube and having a longitudinal axis and a distal tip and having a joint coupling the distant tip to the longitudinal axis.

### SUMMARY OF THE INVENTION

An embodiment of the present invention provides an apparatus as claimed in claim 1, including:
a sheath, consisting of a lumen, having a sheath distal end which is configured to be inserted into a human patient;
a magnetic structure fixedly attached to the sheath distal end;
a probe, having a probe distal end which is configured to be inserted through the lumen into the human patient, the probe including a magnetic transducer which is disposed in the probe distal end and which is configured to generate a signal in response to a magnetic field; and
a processor which is configured to sense a change in the signal due to proximity of the magnetic structure to the transducer.

In a disclosed embodiment the magnetic structure includes a paramagnetic material.

In an alternative disclosed embodiment the magnetic transducer includes a coil, and the change in the signal is in response to a change in inductance of the coil. The inductance may include a self-inductance of the coil, and the magnetic field may be generated by the coil.

In some embodiments the magnetic transducer includes a further coil, and the inductance includes a mutual inductance between the coil and the further coil. The magnetic field may be generated by the further coil.

In a further disclosed embodiment the apparatus includes a force sensor located in the probe distal end and configured to provide an indication of a force on the probe distal end, and the magnetic transducer is an operative component of the force sensor.

In a yet further disclosed embodiment the apparatus includes a position sensor located in the probe distal end and configured to provide an indication of a position of the probe distal end, and the magnetic transducer is an operative component of the position sensor.

Typically, the processor is configured to estimate a distance of the probe distal end from the sheath distal end in response to the change in the signal.

In an alternative embodiment the magnetic structure includes a closed conductive coil.

There is also provided, not forming part of the invention, a method, including:
providing a sheath, including a lumen, having a sheath distal end which is configured to be inserted into a human patient;
fixedly attaching a magnetic structure to the sheath distal end;
inserting a probe, having a probe distal end, through the lumen into the human patient, the probe including a magnetic transducer which is disposed in the probe distal end and which is configured to generate a signal in response to a magnetic field; and
sensing a change in the signal due to proximity of the magnetic structure to the transducer.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for locating the termination of a probe sheath, according to an embodiment of the present invention;
Fig. 2 is a schematic, pictorial view of a catheter in a sheath, according to an embodiment of the present invention;
Fig. 3 is a schematic, sectional view of a distal end of the catheter, according to an embodiment of the present invention;
Fig. 4 is a schematic, sectional view of the catheter distal end and of a sheath distal end, according to an embodiment of the present invention; and
Fig. 5 is a flowchart describing steps for locating a sheath termination with respect to the catheter distal end, according to an embodiment of the present invention.

### DETAILED DESCRIPTION OF EMBODIMENTS

### OVERVIEW

An embodiment of the present invention provides a system for determining the location of the distal end of a probe in relation to the distal end of a sheath being used to guide the probe. The system is typically used in an invasive medical procedure, wherein the distal ends of the probe and the sheath are not accessible, and allows an operator of the system to accurately determine the protrusion of the probe distal end from the sheath distal end. Such a determination, for example, enables the operator to ensure that electrodes on the probe distal end are not obscured by the sheath.

The probe distal end comprises at least one magnetic transducer, which may be an operative component of a force sensor or of a position sensor located in the probe distal end. The at least one magnetic transducer generates a signal in response to a magnetic field impinging on the transducer.

A magnetic structure, typically formed of a paramagnetic material and/or in the shape of a closed conductive coil, is fixed to the sheath distal end. The magnetic structure alters the inductance of the transducers, i.e., the self-inductance of each transducer, as well as the mutual inductance between transducers, depending on the proximity of the sheath distal end to the probe distal end.

The change in inductance causes the signal generated by the magnetic field acting on each transducer to change, and the change is measured by a processor. The processor may use the change to estimate the position of the probe distal end relative to the sheath distal end, and thus determine the protrusion of the probe distal end from the sheath distal end.

### DETAILED DESCRIPTION

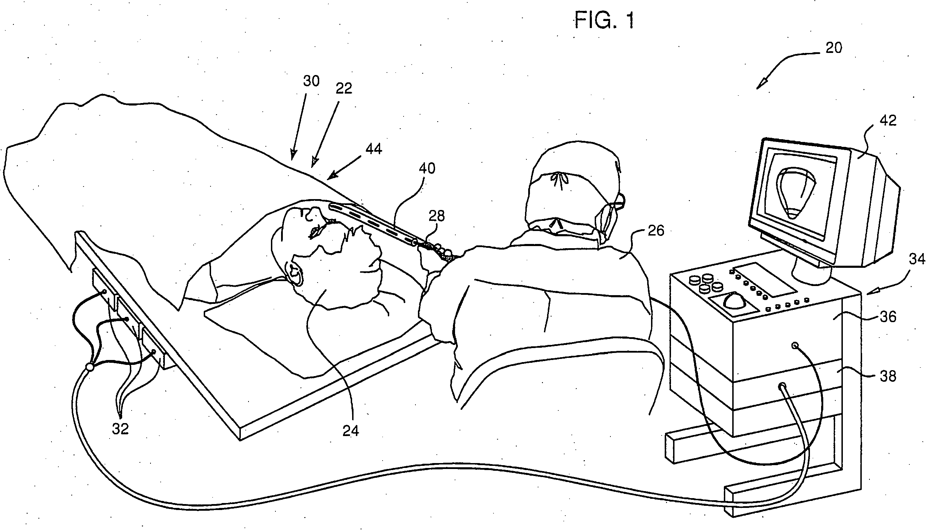

Fig. 1 is a schematic, pictorial illustration of a system 20 for locating the termination of a probe sheath, according to an embodiment of the present invention. System 20 may be based, for example, on the CARTO™ system, produced by Biosense Webster Inc. (Diamond Bar, California). This system comprises an invasive probe in the form of a catheter 28 and a control console 34. In the embodiment described hereinbelow, it is assumed that catheter 28 is used in ablating endocardial tissue using radiofrequency (RF) energy, as is known in the art. Alternatively, the catheter may be used, *mutatis mutandis*, for other therapeutic and/or diagnostic purposes in the heart or in other body organs.

An operator 26, such as a cardiologist, inserts catheter 28 through the vascular system of a patient 24 so that a probe distal end 30 of the catheter enters a chamber of the patient's heart 22. In order to effectively strengthen the catheter, the cardiologist positions the catheter within a sheath 40, which terminates in a sheath distal end 44. Typically, and as assumed in the description herein, the sheath is inserted into the vascular system of the patient before insertion of the catheter into the sheath. Sheath 40 is typically constructed from a reinforced fluoroplastic having a low magnetic susceptibility, for example, the sheath may be formed from polytetrafluoroethylene (PTFE) braided with stainless steel.

The operator advances the catheter so that the distal tip of the catheter engages endocardial tissue at a desired location or locations. Catheter 28 is typically connected by a suitable connector at its proximal end to console 34. The console may comprise a radio frequency (RF) generator, which supplies high-frequency electrical energy via the catheter for ablating tissue in the heart at the locations engaged by the distal tip. Alternatively or additionally, the catheter and system may be configured to perform other therapeutic and diagnostic procedures that are known in the art.

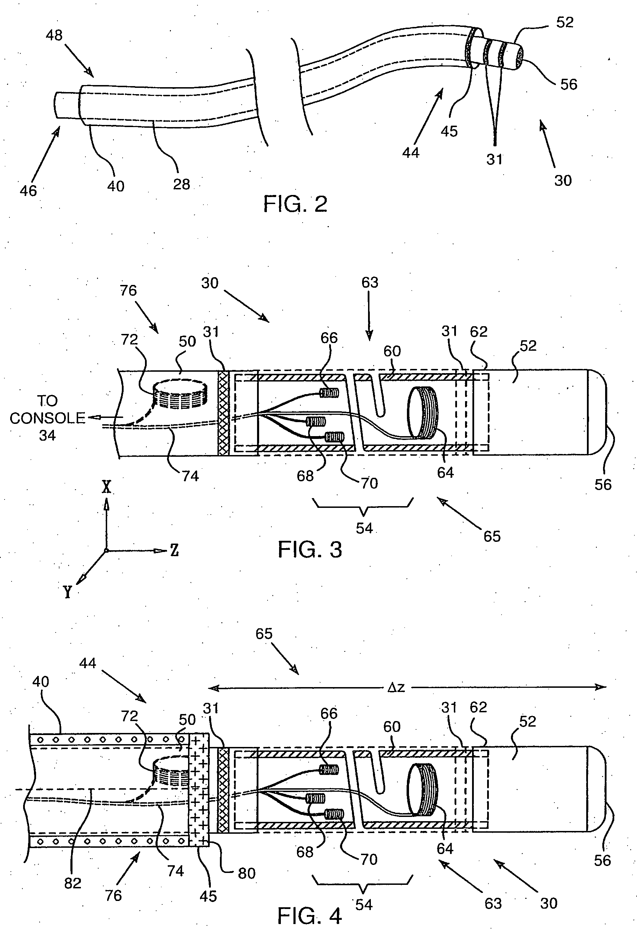

Console 34 may use magnetic position sensing to determine position coordinates of distal end 30 of catheter 28 inside heart 22. For this purpose, a driver circuit 38 in console 34 drives field generators 32 to generate magnetic fields in the vicinity of the body of patient 24. Typically, the field generators comprise coils, which are placed below the patient's torso at known positions external to the patient. These coils generate magnetic fields within the body in a predefined working volume that contains heart 22. A magnetic field sensor within distal end 30 of catheter 28 (shown in Figs. 3 and 4) generates electrical signals in response to these magnetic fields. A signal processor 36 processes these signals in order to determine the position coordinates of the distal end, the position coordinates typically including both location and orientation coordinates. This method of position sensing is implemented in the above-mentioned CARTO system and is described in detail in U.S. Patents 5,391,199, 6,690,963, 6, 484, 118, 6,239,724, 6, 618, 612 and 6, 332, 089.

Alternatively or additionally, console 34 may use other methods known in the art to determine the position coordinates of distal end 30, such methods including, for example, measurements of impedances between the distal end and electrodes on the skin of patient 24. U. S. Patent Applications 11/182,272 filed July 15, 2005 (now issued U.S. Patent No. 7,536,218) and 12/556,639 filed September 10, 2009 (U.S. Patent Publication No. 2010/0079158) describe methods of position sensing using both magnetic field generators and impedance measurements.

Processor 36 typically comprises a general-purpose computer, with suitable front end and interface circuits for receiving signals from catheter 28 and controlling the other components of console 34. The processor may be programmed in software to carry out the functions that are described herein. The software may be downloaded to console 34 in electronic form, over a network, for example, or it may be provided on non-transitory tangible media, such as optical, magnetic or electronic memory media. Alternatively, some or all of the functions of processor 36 may be carried out by dedicated or programmable digital hardware components. Based on the signals received from the catheter and other components of system 20, processor 36 drives a display 42 to give operator 26 visual feedback regarding the position of distal end 30 in the patient's body, and the relation of probe distal end 30 to sheath distal end 44. Display 42 may also provide status information and guidance regarding the procedure that is in progress.

Fig. 2 is a schematic, pictorial view of catheter 28 in sheath 40, according to an embodiment of the present invention. A proximal end 46 of the catheter and a proximal end 48 of the sheath are both able to be manipulated by operator 26, by virtue of being outside the body of patient 24. One or more generally similar ring-like electrodes 31 are mounted on and encircle distal end 30 of the catheter. Herein by way of example there are assumed to be two electrodes 31. In addition, an electrode 56 is formed on a distal tip 52 of catheter 28. A magnetic structure 45, described in more detail below with reference to Fig. 4, is mounted on sheath distal end 44, in proximity to the sheath distal end termination. As illustrated in Fig. 2, and as explained in more detail with respect to Figs. 3, 4, and 5, by manipulation of their proximal ends, operator 26 is able adjust the position of sheath distal end 44 relative to probe distal end 30 so that the sheath encloses a portion of the probe, so as to strengthen catheter 28 while not covering electrodes 31 and 56.

Fig. 3 is a schematic, sectional view of distal end 30 of catheter 28, showing details of the structure of a force sensor 65 at the distal end, according to an embodiment of the present invention. For clarity in the following description, distal end 30 is assumed to be generally cylindrical, and is assumed to define an orthogonal set of axes, with the cylindrical axis being parallel to the z-axis. The catheter comprises an insertion tube 50, which is typically inserted into the heart percutaneously through a blood vessel, such as the vena cava or the aorta. Typically, electrode 56 on distal tip 52 of the catheter engages endocardial tissue (for simplicity the tissue is not shown in the diagram). Alternatively or additionally, at least some of electrodes 31 may contact the endocardial tissue.

Insertion tube 50 is connected to distal tip 52 by an elastic joint 54 which comprises a resilient coupling member 60. In an exemplary embodiment, the coupling member has the form of a tubular piece of an elastic material, with a helical cut along a portion of its length. For example, the coupling member may comprise a superelastic alloy, such as nickel titanium (Nitinol). The helical cut causes the tubular piece to behave like a spring in response to forces exerted on distal tip 52. Further details regarding the fabrication and characteristics of this sort of coupling member are presented in U.S. Patent Applications 12/134,592 filed June 6, 2008 (will issue on May 15, 2012 as U.S. Patent No. 8,180,431) and 12/327,226 filed on December 3, 2008 (U.S. Publication No. 2009/0138007).

Alternatively, the coupling member has a plurality of helical cuts in a portion of its length, such as is described in U.S. Patent Application 12/627,327 filed on November 30, 2009 (U.S. Publication No. 2011/0130648). Further alternatively, the coupling member may comprise a coil spring or any other suitable sort of resilient component with the desired flexibility and strength characteristics.

The insertion tube is covered by a flexible, insulating material 62, such as Celcon®, Teflon®, or heat-resistant polyurethane, for example. The area of joint 54 is covered, as well, by a flexible, insulating material, which may be the same as material 62 or may be specially adapted to permit unimpeded bending and compression of the joint. (This material is shown cut away in Fig. 3 in order to expose the internal structure of the catheter.) Distal tip 52 may be covered, at least in part, by electrode 56, and material 62 may be encircled by electrodes 31. Electrodes 31 and 56 are typically made of a conductive material, such as a platinum/iridium alloy. Alternatively, other suitable materials may be used, as will be apparent to those skilled in the art. Further alternatively, for some applications, the distal tip may be made without a covering electrode, and/or electrodes 31 may not be present. The distal tip is typically relatively rigid, by comparison with the flexible insertion tube.

Coupling member 60 is configured to permit axial displacement (i.e., lateral movement along the z-axis) and angular deflection of the distal tip in x and/or y directions, in proportion to the force on the tip. Measurement of the displacement and deflection by processor 36 thus gives an indication of the force on the tip. The force indication may be used by the operator of catheter 20 is ensuring that the distal tip is pressing against the endocardium firmly enough to give the desired therapeutic or diagnostic result, but not so hard as to cause undesired tissue damage.

A joint sensing assembly 63, comprising coils 64, 66, 68 and 70 within catheter 28, provides accurate reading of the position of distal tip 52 relative to the distal end of insertion tube 50, including axial displacement and angular deflection. These coils are one type of magnetic transducer that may be used in embodiments of the present invention, and act as operative components of force sensor 65, as explained below. A "magnetic transducer," in the context of the present patent application and in the claims, means a device that generates a magnetic field in response to an applied electrical current and/or outputs an electrical signal in response to an applied magnetic field. Although the embodiments described herein use coils as magnetic transducers, other types of magnetic transducers may be used in alternative embodiments, as will be apparent to those skilled in the art.

The coils in assembly 63 are divided between two subassemblies on opposite sides of joint 54: One subassembly comprises coil 64, which is driven by a current via a cable 74 from console 34 to generate a magnetic field. This field is received by a second subassembly, comprising coils 66, 68 and 70, which are located in a section of the catheter that is spaced axially apart from coil 64. (The term "axial," as used in the context of the present patent application and in the claims, refers to the direction of the longitudinal axis of distal end 30 of catheter 28, which is identified as the z-direction in Fig. 3. An axial plane is a plane perpendicular to this longitudinal axis, and an axial section is a portion of the catheter contained between two axial planes.) Coils 66, 68 and 70 generate electrical signals in response to the magnetic field generated by coil 64. These signals are conveyed by cable 74 to processor 36, which processes the signals in order to measure the axial displacement and angular deflection of joint 54.

Coils 66, 68 and 70 are fixed in catheter 28 at different radial locations. (The term "radial" refers to coordinates relative to the catheter axis, i.e., coordinates in an X-Y plane in Fig. 3.) Specifically, in this embodiment, coils 66, 68 and 70 are all located in the same axial plane at different azimuthal angles about the catheter axis. For example, the three coils may be spaced azimuthally 120° apart at the same radial distance from the axis.

The axes of coils 64, 66, 68 and 70 are parallel to the catheter axis (and thus to one another, as long as joint 54 is undeflected). Consequently, coils 66, 68 and 70 will output strong signals in response to the field generated by coil. 64, and the signals will vary strongly with the distances of coils 66, 68 and 70 from coil 64. (Alternatively, the axis of coil 64 and/or coils 66, 68 and 70 may be angled relative to the catheter axis, as long as the coil axes have a sufficient parallel component in order to give substantial signals.) Angular deflection of tip 52 will give rise to a differential change in the signals output by coils 66, 68 and 70, depending on the direction and magnitude of deflection, since one or two of these coils will move relatively closer to coil 64. Compressive displacement of the tip will give rise to an increase in the signals from all of coils 66, 68 and 70.

Processor 36 analyzes the signals output by coils 66, 68 and 70 in order to measure the deflection and displacement of joint 54. The sum of the changes in the signals gives a measure of the compression, while the difference of the changes gives a measure of the deflection. The vector direction of the difference gives an indication of the bend direction. A suitable calibration procedure may be used to measure the precise dependence of the signals on deflection and displacement of the joint.

Various other configurations of the coils in the sensing subassemblies may also be used, in addition to the configuration shown and described above. For example, the positions of the subassemblies may be reversed, so that the field generator coil is on the proximal side of joint 54, and the sensor coils are in the distal tip. As another alternative, coils 66, 68 and 70 may be driven as field generators (using time- and/or frequency-multiplexing to distinguish the fields), while coil 64 serves as the sensor. The sizes and numbers of the coils in Fig. 3 are shown only by way of example, and larger or smaller numbers of coils may similarly be used, in various different positions. Typically, one of the subassemblies comprises at least two coils, in different radial positions, to allow differential measurement of joint deflection. However, in some embodiments measuring joint compression, each subassembly comprises only one coil.

Joint sensing assembly 63, together with elastic joint 54, acts as force sensor 65, being able to measure both the direction and the magnitude of the force acting on tip 52. Force sensor 65 may also act as a pressure sensor, assuming that an area of tip 52 to which the force is applied is known or can be estimated.

As described below, in a force sensor part of a prior calibration of the force sensor, processor 36 determines a relation between the force on tip 52 and the coil signals of the assembly, the coil signals indicating movement of joint 54. Another part of the calibration, relating the position of sheath 40 to the sensing assembly is also described below, with reference to Fig. 4.

One or more of coils 64, 66, 68 and 70 may also be used to output signals in response to the magnetic fields generated by field generators 32, and thus serve as position sensing coils. Processor 36 processes these signals in order to determine the coordinates (position and orientation) of distal end 30 in the external frame of reference that is defined by the field generators. Additionally or alternatively, one or more further coils 72 (or other magnetic sensors) acting as operative components of a position sensor 76 may be deployed in the distal end of the catheter for this purpose. The position sensing coils in distal end 30 of catheter 28 enable console 34 to output both the location and orientation of the catheter in the body and the displacement and deflection of tip 52, as well as the force on the tip.

Fig. 4 is a schematic, sectional view of probe distal end 30 and of sheath distal end 44, according to an embodiment of the present invention. Sheath distal end 44 of sheath 40 terminates at a sheath termination 80, and magnetic structure 45 is mounted on the sheath distal end in proximity to the termination, typically at the termination. In one embodiment structure 45 is in the form of a closed conductive coil or ring that encircles the sheath at its distal end, so that the structure interacts with a magnetic field generated by or coupled to coils in the probe distal end, such as coils 66, 68, and 70. Alternatively, structure 45 may comprise one or more elements mounted on sheath distal end 44 that are galvanically insulated from each other. Typically, structure 45 is substantially symmetrical with respect to an axis 82 of the sheath.

The material of structure 45 may be selected so that its magnetic properties cause it to interact with a magnetic field such as that described above. Typically the material is an inert, bio-compatible material that is paramagnetic, with a relatively large magnetic susceptibility. The material may comprise an element or a compound. In one embodiment, structure 45 is formed from platinum.

The magnetic interaction between magnetic structure 45 and the coils in the probe distal end changes the self-inductance of each coil, as well as the mutual inductance between the coils. For a given coil the change in self-inductance is a function of the distance between the structure and the coil, as well as of the relative orientation of the structure and the coil. For a given pair of coils, the change in mutual induction is a function of the distances between the structure and each of the coils, and of the relative orientations of the structure with each of the coils. Typically, the change in self-inductance or mutual inductance is relatively large if the axis of structure 45 is parallel to the axis of the coil or coils, and is relatively small if the axis of the structure is orthogonal to the axis of the coils.

Thus, introduction of the distal sheath end, with its attached magnetic structure 45, into the vicinity of the joint sensing assembly alters the self-inductance of each of the coils in the assembly, as well as the mutual inductance between each of the coils. The changes in the self-inductance and the mutual inductance are a function of the proximity of the magnetic structure to the coil or coils being considered.

For coils such as coils 66, 68, and 70 that are fixed in relation to each other in the distal end, a change in mutual inductance between any two of these coils may be used to measure proximity of structure 45. Alternatively or additionally, a change in the self inductance of any of the coils in the distal end may be used to measure the proximity. The proximity may be quantified as a distance Δz of sheath termination 80 from an arbitrary point on the distal end. By way of example, and as illustrated in Fig. 4, distance Δz is assumed to be measured to the distal end of distal tip 52.

In a sheath location part of the calibration procedure referred to above, the change of inductance, self and/or mutual, for one or more of the coils at distal end 30 of the probe is measured for different values of distance Δz. The self-inductance for a given coil may be measured by injecting a signal of known amplitude and frequency into the coil, and determining an amplitude and/or a phase of the current generated. From the generated current and injected amplitude, and allowing for a DC resistance of the coil, the self-inductance may be determined. The mutual inductance for a given pair of coils may be found in a similar manner, by injecting a signal of known amplitude and frequency into a first coil of the pair, and determining the current produced in the second coil.

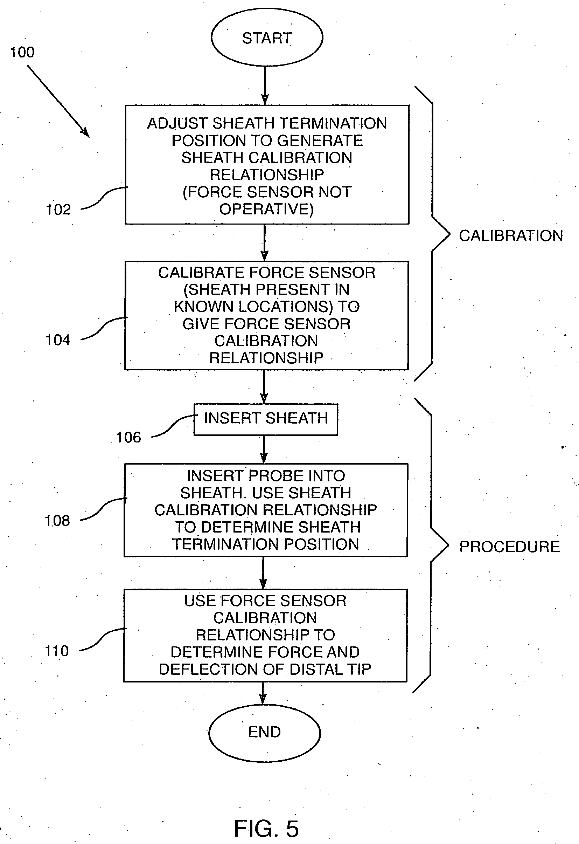

Fig. 5 is a flowchart 100 describing steps for locating sheath termination 80 with respect to distal end 30, according to an embodiment of the present invention. The description assumes the presence of a force sensor, with magnetic transducers, corresponding to joint sensing assembly 63, wherein coil 64 acts as a magnetic field transmitter. Those having ordinary skill in the art will be able to adapt the description, *mutatis mutandis,* for the presence of magnetic transducers other than those of a force sensor, such as coordinate sensing coil 72.

In a first calibration step 102, processor 36 implements the sheath location part of the calibration procedure, which by way of example, is assumed to use changes of mutual inductance. Probe 28 is inserted into sheath 40, generally as illustrated in Fig. 2, but with distal ends 44 and 30 outside the body of patient 24 so that distance Δz may be independently measured. A sheath calibration signal of known amplitude and frequency, herein termed the sheath location frequency, is injected into coil 66, and the processor measures the signals generated by coils 68 and 70, caused respectively by the mutual inductance between the pair of coils 66 and 68, and the pair of coils 66 and 70. The processor records the changes in signals in coils 68 and 70 for different values of distance Δz of the sheath termination, and forms a sheath location calibration relationship, typically using interpolation, between the signal changes and distances Δz. The processor stores the calibration relationship for use during a procedure involving sheath 40 and probe 28. The changes in signals are typically calculated as differences from the signals in coils 68 and 70 when the sheath termination is not close to joint sensing assembly 63, so that it does not influence the signals generated in coils 68 and 70. During step 102, coil 64 is typically inoperative.

In a second calibration step 104, processor 36 implements the force sensor part of the calibration procedure described above. I.e., the processor injects a force calibration signal of known amplitude and frequency, herein termed the force sensor frequency, into coil 64. The processor then measures signals from coils 66, 68, and 70 for known forces and deflections of distal tip 52, and compiles relations between the signals and the forces and deflections. The procedure for step 104 is repeated for different positions of sheath termination 80, i.e., for different values of distance Δz. One of the sets of relations is for the case when magnetic structure 45 does not affect the signals in coils 66, 68, and 70, i.e., for a value of Δt that is large.

For each different value of distance Δz there is a set of relations between the coil signals and the forces and deflections of the distal tip. Typically, the processor uses interpolation in order to generate a force sensor calibration relationship between sets of coil signals, the forces and deflections on distal tip 52, and values of distance Δz.

Steps 102 and 104 constitute a calibration section of flowchart 100. Typically, the sheath location frequency and the force sensor frequency are different from each other, and also from other frequencies, such as the ablating frequency and the field generator frequencies, used during the procedure. Using different frequencies enables each coil in distal end 30 to perform multiple functions simultaneously, as well as reducing interference, such as may occur during tissue ablation.

In an initiate procedure step 106, sheath 40 is inserted into patient 24 so that the distal end of the sheath is in the general area of the endocardial tissue to be ablated.

In a probe insertion step 108, probe 28 is inserted into sheath 40, until distal tip 52 contacts the endocardial tissue to be ablated. During the insertion, processor 36 injects the sheath calibration signal into coil 66, and measures the resulting signals generated in coils 68 and 70. The processor compares the measured signals with the sheath location calibration relationship determined in step 102. From the comparison, processor 36 estimates the position of sheath termination 80, corresponding to measuring the value of distance Δz. The processor may present the distance, in a graphical and/or text format, on display 42. Typically, using the information on the display, operator 26 may manipulate the proximal ends of the sheath and probe to achieve a desired protrusion of probe distal end 44 from sheath termination 80.

In a force determination step 110, the processor injects the force calibration signal into coil 64, and measures the resulting signals generated in coils 66, 68, and 70. From the signals, and knowing the position of sheath termination 80 determined in step 108, the processor uses the force sensor calibration relationship to determine the force and deflection of distal tip 52.

Consideration of flowchart 100 illustrates that not only is system 20 able to achieve a desired protrusion of distal end 44 from termination 80, but the system is also able to allow for any changes in signals generated by the coils of assembly 63 due to the proximity of magnetic structure 45.

It will be understood that the description of flowchart 100, assuming the presence of magnetic transducers in a force sensor in distal end 30, is by way of example. In an alternative embodiment, rather than using transducers which are part of a force sensor, other transducers, such as coils in distal end 30 which are used to determine the coordinates of the distal end, may be used. It will also be understood that using signal changes due to the proximity of structure 45 changing the mutual inductance between magnetic transducers in distal end 30 is by way of example, and that signal changes due to the change in self-inductance in one or more magnetic transducers in the distal end may be used in place of, or in addition to, the changes caused by the change in mutual inductance.

### Aspects not yet claimed:

Aspect 1. A method, comprising:
   providing a sheath, comprising a lumen, having a sheath distal end which is configured to be inserted into a human patient;
   fixedly attaching a magnetic structure to the sheath distal end;
   inserting a probe, having a probe distal end, through the lumen into the human patient, the probe comprising a magnetic transducer which is disposed in the probe distal end and which is configured to generate a signal in response to a magnetic field; and
   sensing a change in the signal due to proximity of the magnetic structure to the transducer.
Aspect 2. The method according to aspect 1, wherein the magnetic structure comprises a paramagnetic material.
Aspect 3. The method according to aspect 1, wherein the magnetic transducer comprises a coil, and wherein the change in the signal is in response to a change in inductance of the coil.
Aspect 4. The method according to aspect 3, wherein the inductance comprises a self-inductance of the coil.
Aspect 5. The method according to aspect 4, wherein the magnetic field is generated by the coil.
Aspect 6. The method according to aspect 3, wherein the magnetic transducer comprises a further coil, and wherein the inductance comprises a mutual inductance between the coil and the further coil.
Aspect 7. The method according to aspect 6, wherein the magnetic field is generated by the further coil.
Aspect 8. The method according to aspect 1, and comprising locating a force sensor in the probe distal end and configuring the force sensor to provide an indication of a force on the probe distal end, wherein the magnetic transducer is an operative component of the force sensor.
Aspect 9. The method according to aspect 1, and comprising locating a position sensor in the probe distal end and configuring the position sensor to provide an indication of a position of the probe distal end, wherein the magnetic transducer is an operative component of the position sensor.
Aspect 10. The method according to aspect 1, and comprising estimating a distance of the probe distal end from the sheath distal end in response to the change in the signal.
Aspect 11. The method according to aspect 1, wherein the magnetic structure comprises a closed conductive coil.

## Claims

1. Apparatus, comprising:
a probe (28), having a probe distal end (52) and comprising a magnetic transducer (66, 68, 70) which is disposed in the probe distal end (52) and which is configured to generate a signal in response to a magnetic field;
**characterised in that** the apparatus further comprises:
a sheath (40), comprising a lumen, having a sheath distal end (44) which is configured to be inserted into a human patient;
a magnetic structure (45) fixedly attached to the sheath distal end; and
a processor which is configured to sense a change in the signal due to proximity of the magnetic structure to the transducer;
wherein the probe is configured to be inserted through the lumen into a human patient.

2. The apparatus according to claim 1, wherein the magnetic structure (45) comprises a paramagnetic material.

3. The apparatus according to claim 1, wherein the magnetic transducer (66, 68, 70) comprises a coil, and wherein the change in the signal is in response to a change in inductance of the coil.

4. The apparatus according to claim 3, wherein the inductance comprises a self-inductance of the coil.

5. The apparatus according to claim 4, wherein the magnetic field is generated by the coil.

6. The apparatus according to claim 3, wherein the magnetic transducer (66, 68, 70) comprises a further coil, and wherein the inductance comprises a mutual inductance between the coil and the further coil.

7. The apparatus according to claim 6, wherein the magnetic field is generated by the further coil.

8. The apparatus according to claim 1, and comprising a force sensor (65) located in the probe distal end (52) and configured to provide an indication of a force on the probe distal end (52), and wherein the magnetic transducer (66, 68, 70) is an operative component of the force sensor.

9. The apparatus according to claim 1, and comprising a position sensor (76) located in the probe distal end (52) and configured to provide an indication of a position of the probe distal end (52), and wherein the magnetic transducer (66, 68, 70) is an operative component of the position sensor.

10. The apparatus according to claim 1, wherein the processor is configured to estimate a distance of the probe distal end (52) from the sheath distal end (44) in response to the change in the signal.

11. The apparatus according to claim 1, wherein the magnetic structure (45) comprises a closed conductive coil.

## Patentansprüche

1. Vorrichtung, umfassend:
eine Sonde (28) mit einem distalen Ende der Sonde (52) und umfassend einen Magnetwandler (66, 68, 70), der in dem distalen Ende der Sonde (52) angeordnet ist und der dafür gestaltet ist, ein Signal in Antwort auf ein Magnetfeld zu erzeugen;
**dadurch gekennzeichnet, dass** die Vorrichtung ferner umfasst:
eine Hülle (40), umfassend ein Lumen, mit einem distalen Ende der Hülle (44), das dafür gestaltet ist, in einen menschlichen Patienten eingeführt zu werden;
eine magnetische Struktur (45), die fest an dem distalen Ende der Hülle befestigt ist; und
einen Prozessor, der dafür gestaltet ist, eine Veränderung des Signals aufgrund von Annäherung der magnetischen Struktur an den Wandler wahrzunehmen;
wobei die Sonde dafür gestaltet ist, durch das Lumen in einen menschlichen Patienten eingeführt zu werden.

2. Vorrichtung gemäß Anspruch 1, wobei die magnetische Struktur (45) ein paramagnetisches Material umfasst.

3. Vorrichtung gemäß Anspruch 1, wobei der Magnetwandler (66, 68, 70) eine Spule umfasst und wobei die Veränderung des Signals in Antwort auf eine Veränderung der Induktivität der Spule erfolgt.

4. Vorrichtung gemäß Anspruch 3, wobei die Induktivität eine Selbstinduktivität der Spule umfasst.

5. Vorrichtung gemäß Anspruch 4, wobei das Magnetfeld durch die Spule erzeugt wird.

6. Vorrichtung gemäß Anspruch 3, wobei der Magnetwandler (66, 68, 70) eine weitere Spule umfasst und wobei die Induktivität eine Gegeninduktivität zwischen der Spule und der weiteren Spule umfasst.

7. Vorrichtung gemäß Anspruch 6, wobei das Magnetfeld durch die weitere Spule erzeugt wird.

8. Vorrichtung gemäß Anspruch 1, umfassend einen Kraftsensor (65), der in dem distalen Ende der Sonde (52) angeordnet ist und dafür gestaltet ist, eine Meldung über eine Kraft an dem distalen Ende der Sonde (52) zu liefern, und wobei der Magnetwandler (66, 68, 70) eine funktionelle Komponente des Kraftsensors ist.

9. Vorrichtung gemäß Anspruch 1, umfassend einen Positionssensor (76), der in dem distalen Ende der Sonde (52) angeordnet ist und dafür gestaltet ist, eine Meldung über die Position des distalen Endes der Sonde (52) zu liefern, und wobei der Magnetwandler (66, 68, 70) eine funktionelle Komponente des Positionssensors ist.

10. Vorrichtung gemäß Anspruch 1, wobei der Prozessor dafür gestaltet ist, den Abstand des distalen Endes der Sonde (52) von dem distalen Ende der Hülle (44) in Antwort auf die Veränderung des Signals abzuschätzen.

11. Vorrichtung gemäß Anspruch 1, wobei die magnetische Struktur (45) eine geschlossene leitfähige Spule umfasst.

## Revendications

1. Appareil comprenant :
une sonde (28), comportant une extrémité distale (52) de sonde et comprenant un capteur magnétique (66, 68, 70) qui est disposé dans l'extrémité distale (52) de sonde et qui est configuré pour émettre un signal en réponse à un champ magnétique,
**caractérisé en ce que** l'appareil comprend en outre :
une gaine (40) comprenant une lumière et comportant une extrémité distale (44) de gaine, qui est configurée pour être introduite dans un patient humain ;
une structure magnétique (45) fixée à demeure à l'extrémité distale de gaine ; et
un processeur qui est configuré pour détecter une variation du signal dû à la proximité de la structure magnétique avec le capteur,
dans lequel la sonde est configurée pour être insérée par la lumière dans un patient humain.

2. Appareil selon la revendication 1, dans lequel la structure magnétique (45) comprend un matériau paramagnétique.

3. Appareil selon la revendication 1, dans lequel le capteur magnétique (66, 68, 70) comprend une bobine et dans lequel la variation du signal est en réaction à une variation de l'inductance de la bobine.

4. Appareil selon la revendication 3, dans lequel l'inductance comprend une inductance propre de la bobine.

5. Appareil selon la revendication 4, dans lequel le champ magnétique est produit par la bobine.

6. Appareil selon la revendication 3, dans lequel le capteur magnétique (66, 68, 70) comprend une autre bobine et dans lequel l'inductance comprend une inductance mutuelle entre la bobine et la bobine supplémentaire.

7. Appareil selon la revendication 6, dans lequel le champ magnétique est produit par l'autre bobine.

8. Appareil selon la revendication 1, comprenant un capteur de force (65) situé dans l'extrémité distale (52) de sonde et configuré pour fournir une indication concernant une force s'exerçant sur l'extrémité distale (52) de sonde et dans lequel le capteur magnétique (66, 68, 70) est un composant opérationnel du capteur de force.

9. Appareil selon la revendication 1, comprenant un capteur de position (76) situé dans l'extrémité distale (52) de sonde et configuré pour fournir une indication sur la position de l'extrémité distale (52) de sonde et dans lequel le capteur magnétique (66, 68, 70) est un composant opérationnel du capteur de position.

10. Appareil selon la revendication 1, dans lequel le processeur est configuré pour estimer la distance entre l'extrémité distale (52) de sonde et l'extrémité distale (44) de gaine en réponse à la variation du signal.

11. Appareil selon la revendication 1, dans lequel la structure magnétique (45) comprend des spires conductrices jointives.
